# FASCICULE DE BREVET EUROPEEN

(11) **EP 0 869 946 B1**
(45) Date de publication et mention de la délivrance du brevet: **31.10.2001**
(21) Numéro de dépôt: 96942431.6
(22) Date de dépôt: 19.12.1996
(51) Int. Cl.: C07D 233/72, A61K 31/415

(54) **PHENYLIMIDAZOLIDINES ET LEUR UTILISATION COMME AGENT ANTI-ANDROGENE**
PHENYLIMIDAZOLIDINE UND DEREN VERWENDUNG ALS ANTI-ANDROGENE MITTEL
PHENYLIMIDAZOLIDINES AND USE THEREOF AS AN ANTIANDROGENIC AGENT

(30) Priorité: 22.12.1995 FR 9515323
(43) Date de publication de la demande: 14.10.1998
(73) Titulaire: Aventis Pharma S.A., 92160 Antony (FR)
(72) Inventeur: CLAUSSNER, André, F-93250 Villemomble (FR); GOUBET, François, F-75015 Paris (FR); TEUTSCH, Jean-Georges, F-93500 Pantin (FR)
(86) Numéro de dépôt international: FR9602029
(87) Numéro de publication internationale: WO9723464

(56) Documents cités:
- WO-A-95/18794
- FR-A- 2 693 461

## Description

La présente invention concerne des phénylimidazolidines comportant notamment un radical nitrooxy ou carbonyloxy, leur procédé de préparation, les nouveaux intermédiaires obtenus, leur application à titre de médicaments, leur nouvelle utilisation et les compositions pharmaceutiques les renfermant.

Les documents WO-A-95/18794 et FR-A-2 693 461 décrivent des dérivés phénylimidazolidines présentant une activité anti-androgène.

La présente invention a pour objet les produits de formule (I) 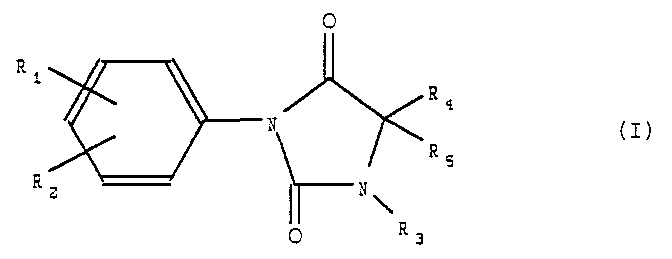 dans laquel R₁ et R₂ représentent un radical cyano et un radical trifluorométhyle,
R₃ représente un radical alkyle, alkényle ou alkynyle, linéaires ou ramifiés, renfermant au plus 4 atomes de carbone substitué par un radical choisi parmi les radicaux nitrooxy et 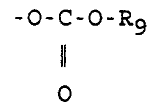 dans lequel R₉ représente, un radical alkyle, linéaire ou ramifié, renfermant au plus 4 atomes de carbone ou un radical cycloalkyle renfermant 5 ou 6 chaînons,
R₄ et R₅ soit, identiques ou différents, représentent un radical méthyle éventuellement substitué par un atome de fluor, soit forment avec l'atome de carbone auquel ils sont attachés un radical cyclohexyle
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

Dans les produits de formule (I) et dans ce qui suit :
- le terme halogène désigne les atomes de fluor, de chlore, de brome ou d'iode.
   On préfère les atomes de fluor, de chlore ou de brome.
- le terme radical alkyle linéaire ou ramifié désigne les radicaux méthyle, éthyle, propyle, isopropyle, butyle, isobutyle, sec-butyle, tert-butyle, ainsi que leurs isomères de position linéaires ou ramifiés.
   On préfère les radicaux alkyle ayant au plus 4 atomes de carbone et notamment les radicaux méthyle, éthyle, propyle, isopropyle, n-butyle.
- le terme radical alkényle linéaire ou ramifié désigne les radicaux vinyle, allyle, 1-propényle, butényle, 1-butényle, ainsi que leurs isomères de position linéaires ou ramifiés.
   Parmi les radicaux alkényle, on préfère les valeurs vinyle, allyle, n-butényle ou isobutényle,
- le terme alkynyle désigne un radical linéaire ou ramifié ayant au plus 4 atomes de carbone tel que par exemple éthynyle, propargyle, butynyle.
   Parmi les radicaux alkynyle, on préfère le radical propargyle.

Des exemples de groupement protecteur du radical hydroxyle protégé sont donnés notamment dans le livre usuel de l'homme du métier : Protective Groups in Organic Synthesis, Theodora W. Greene, Harvard University, imprimé en 1981 par Wiley-Interscience Publishers, John Wiley & Sons.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoique, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique, acide métasulfobenzoïque et les acides aryldisulfoniques.

On peut citer plus particulièrement les sels formés avec les acides chlorhydrique ou méthanesulfonique par exemple.

On peut rappeler que la stéréoisomérie peut être définie comme l'isomérie de composés ayant mêmes formules développées, mais dont les différents groupes sont disposés différemment dans l'espace, tels que notamment dans les formes bateau et chaise du cyclohexane et des cyclohexanes mono-substitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

Dans les produits de formule (I) et dans ce qui suit, on peut noter que :
- les atomes d'hydrogène, que renferment les radicaux alkyle ou alkényle éventuellement substitués que peut représenter R₃, peuvent être des atomes de deutérium,
- les atomes de fluor, que peuvent représenter les atomes d'halogène, peuvent être un atome ¹⁸F utile pour l'imagerie médicale.

La présente invention a notamment pour objet les produits de formule (I) telle que définie ci-dessus dans laquelle R₄ et R₅ représentent un radical méthyle et R₁, R₂, R₃, X et Y ont les significations indiquées ci-dessus.

Parmi les produits préférés de l'invention, on peut citer plus précisément les produits de formule (I) telle que définie ci-dessus dont les noms suivent :
- carbonate de 4-(3-(4-cyano-3-(trifluorométhyl)phényl)-5,5-diméthyl-2,4-dioxo-1-imidazolidinyl)butyle et de 1-méthyl-éthyle,
- 4-(4,4-diméthyl-2,5-dioxo-3-(4-nitrooxybutyl)-1 imidazolidinyl)-2-(trifluorométhyl)benzonitrile,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

La présente invention a aussi pour objet un procédé de préparation des produits de formule (I), telle que définie ci-dessus, caractérisé en ce que :
soit l'on fait agir en présence d'une base tertiaire un produit de formule (II) : 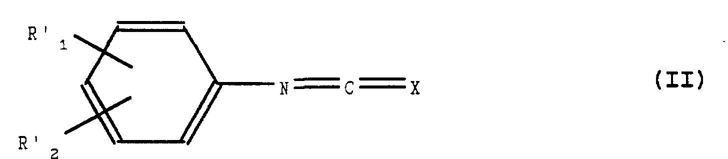 dans laquelle R'₁ et R'₂ ont les significations indiquées ci-dessus, respectivement pour R₁ et R₂, dans lesquelles les éventuelles fonctions réactives sont éventuellement protégées et X représente un atome d'oxygène ou de soufre avec un produit de formule (III) : 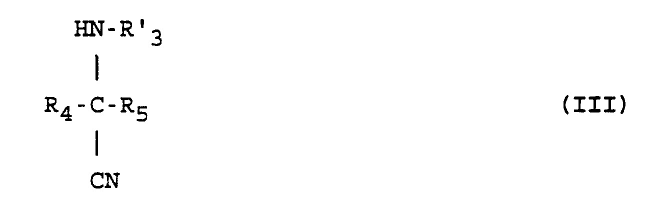 dans laquelle R₄ et R₅ ont les significations indiquées ci-dessus, et R'₃ a la signification indiquée ci-dessus pour R₃, dans lesquelles les éventuelles fonctions réactives sont éventuellement protégées, pour obtenir un produit de formule (IV) : 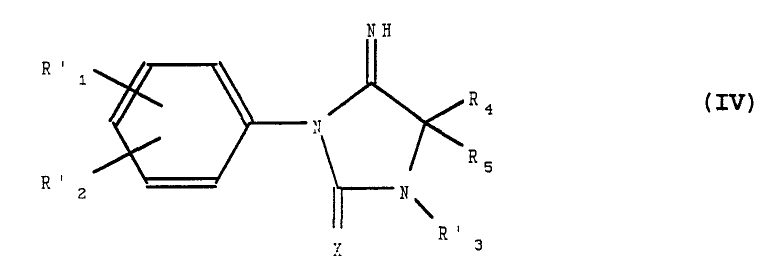 dans laquelle X, R'₁, R'₂, R'₃, R₄ et R₅ ont les significations indiquées ci-dessus,
que l'on transforme en un produit de formule (V) : 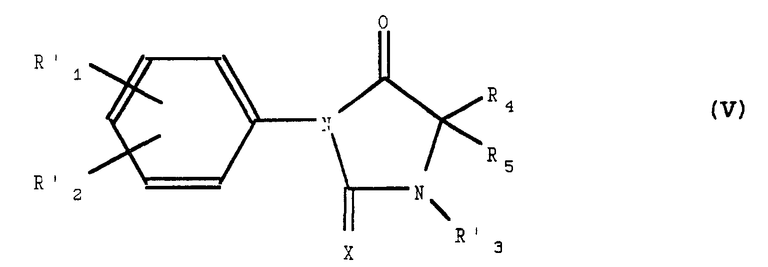 dans laquelle X, R'₁, R'₂, R'₃, R₄ et R₅ ont les significations indiquées ci-dessus,
soit l'on fait agir un produit de formule (VI) : 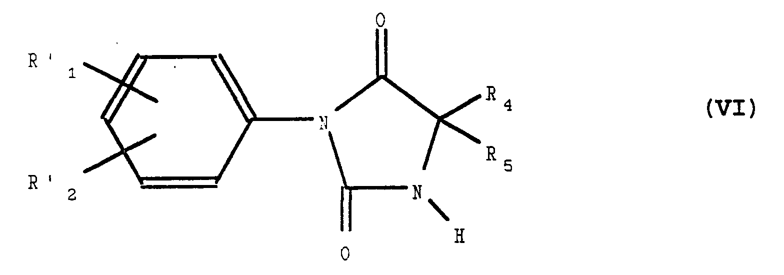 dans laquelle R'₁, R'₂, R₄ et R₅ ont les significations indiquées ci-dessus, avec un réactif de formule Hal-R'₃ dans laquelle R'₃ a la signification indiquée ci-dessus, et Hal représente un atome d'halogène pour obtenir les produits correspondants de formule (V) telle que définie ci-dessus dans laquelle X représente un atome d'oxygène,
soit l'on fait agir sur un produit de formule (VII) : 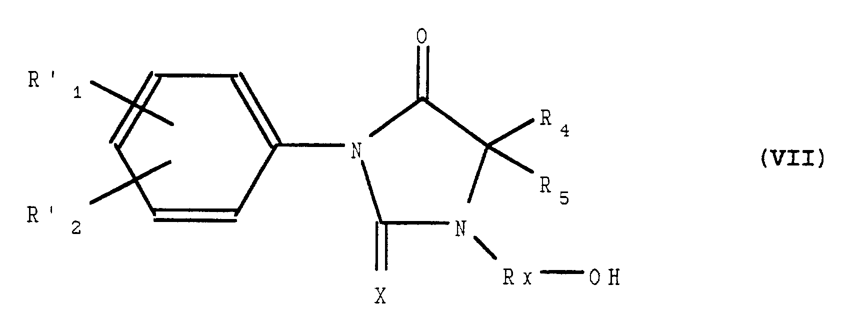 dans laquelle R'₁, R'₂, R₄, R₅ et X ont les significations indiquées ci-dessus,
et Rx représente un radical alkyle, alkényle ou alkynyle, linéaires ou ramifiés, renfermant au plus 4 atomes de carbone, que l'on soumet :
**ou bien** à l'action d'un composé de formule (VIII) : 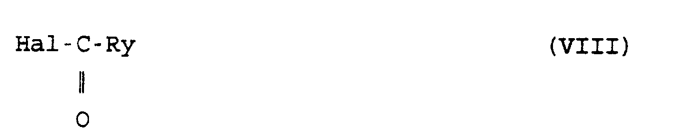 dans laquelle Hal représente un atome d'halogène et Ry représente un radical choisi parmi les radicaux alcoxy, cycloalcoxy, tels que définis ci-dessus, pour obtenir un produit de formule (IX) : 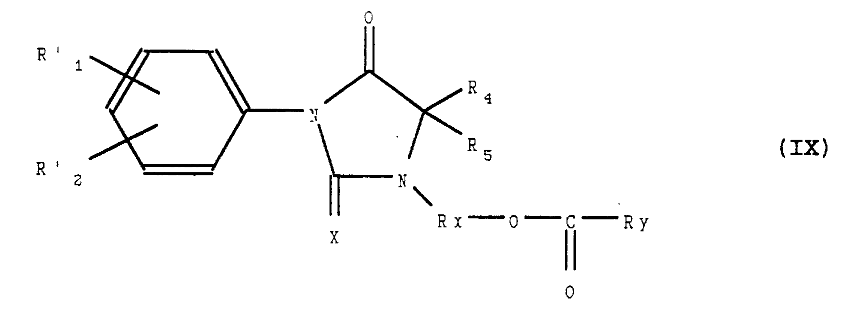 dans laquelle R'₁, R'₂, R₄, R₅, X, Rx et Ry ont les significations indiquées ci-dessus,
**ou bien** à une réaction d'halogénation pour obtenir un produit de formule (X) : 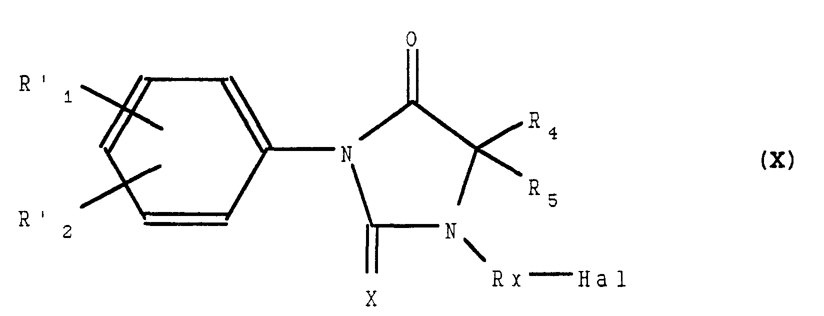 dans laquelle R'₁, R'₂, R₄, R₅, X, Rx et Hal ont les significations indiquées ci-dessus,
que l'on soumet à l'action de nitrate d'argent pour obtenir le produit de formule (XI) : 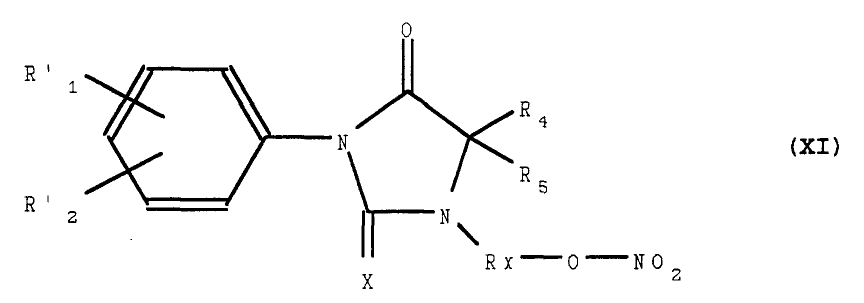 dans laquelle R'₁, R'₂, R₄, R₅, X, Rx et Hal ont les significations indiquées ci-dessus,
produits de formules (IV), (V), (IX) et (XI) que, si nécessaire ou si désiré, pour obtenir des produits de formule (I) telle que définie à la revendication 1, l'on peut soumettre à l'une quelconque ou plusieurs des réactions suivantes, dans un ordre quelconque :
a) le cas échéant transformation du groupement >C=S que peut représenter >C=X en groupement >C=O,
b) libération du radical OH que peut porter R'₃,
c) réaction d'estérification ou éthérification du radical OH que peut porter R₃,
d) réaction d'élimination des éventuels groupements protecteurs,
e) le cas échéant action d'un agent d'estérification, d'amidification ou de salification,
f) réaction de dédoublement des formes racémiques,
   lesdits produits de formule (I) ainsi obtenus étant sous toutes les formes isomères possibles racémiques, énantiomères et diastéréoisomères.

L'action des produits de formule (II) avec les produits de formule (III) pour obtenir les produits de formule (IV) peut être effectuée en présence de chlorure de méthylène, à une température d'environ -30°C, ou encore dans un solvant organique tel que le tétrahydrofuranne ou le dichloroéthane, mais on peut également utiliser l'éther éthylique ou l'éther isopropylique.

On opère en présence d'une base tertiaire telle que la triéthylamine ou encore la pyridine ou la méthyléthylpyridine.

Les éventuelles fonctions réactives qui sont éventuellement protégées, peuvent être notamment les fonctions hydroxy. On utilise pour protéger ces fonctions des groupements protecteurs usuels comme indiqué ci-dessus. On peut citer par exemple et de façon non exhaustive, les radicaux tétrahydropyrannyle, triméthylsilyle, triphénylméthyle ou tert-butyl diméthylsilyle ou encore les groupements protecteurs, connus dans la chimie des peptides comme indiqué par exemple dans le brevet français BF 2.499.995 dont le contenu est incorporé ici par référence.

Dans le produit de formule (III), R₄ et R₅ peuvent former avec l'atome de carbone qui les porte un radical cyclohexyle.

Les réactions éventuelles d'élimination des groupements protecteurs sont effectuées selon les méthodes usuelles connues de l'homme du métier ou, par exemple, comme indiqué dans le brevet BF 2.499.995. Le mode préféré d'élimination est l'hydrolyse acide à l'aide des acides choisis parmi les acides chlorhydrique, benzène sulfonique ou para toluène sulfonique, formique ou trifluoroacétique. On préfère l'acide chlorhydrique.

La réaction éventuelle d'hydrolyse du groupement >C=NH en groupement carbonyle pour transformer notamment le produit de formule (IV) en produit de formule (V) est effectuée de préférence à l'aide d'un acide tel que l'acide chlorhydrique aqueux, par exemple au reflux.

L'action sur les produits de formule (VI) du réactif de formule Hal-R'₃ pour obtenir les produits de formule (V) est effectuée en présence d'une base forte telle que l'hydrure de sodium ou de potassium. On peut opérer par réaction de transfert de phase en présence de sels d'ammonium quaternaires tels que les sels de tétrabutyl ammonium dihydrogénophosphate.

La transformation du radical OH en radical halogène des produits de formule (VII) pour obtenir les produits de formule (X) peut être réalisée dans les conditions usuelles connues de l'homme du métier telles que notamment dans un solvant tel que par exemple le tétrahydrofuranne et action d'un dérivé halogéné tel que notamment, lorsque l'atome d'halogène est un atome de fluor, le diéthylaminotrifluorosulfure (DAST).

On peut également faire agir au préalable de l'anhydride triflique pour obtenir le triflate correspondant que l'on échange ensuite avec le fluorure correspondant ainsi qu'il est décrit ci-après dans les exemples et notamment par action du fluorure de tétrabutylammonium .

Lorsque l'atome d'halogène est un atome de brome, de chlore ou d'iode, on peut agir selon les conditions usuelles connues de l'homme du métier telle que notamment par action, en présence de triphénylphosphine, de l'agent halogénant correspondant tel que par exemple le tétrabromure de carbone, le tétrachlorure de carbone ou encore de l'iode.

Des réactions de transformation du radical OH du produit de formule (VII) pour obtenir les produits de formules (IX) et (XI) telles que définies ci-dessus dans lesquelles R₃ a la signification indiquée ci-dessus, sont données, à titre d'exemple et de façon non exhaustive, dans les préparations des exemples 1 à 4 décrites ci-après.

L'estérification éventuelle du radical OH libre est effectuée dans des conditions classiques. On peut utiliser par exemple un acide ou un dérivé fonctionnel, par exemple un anhydride tel que l'anhydride acétique en présence d'une base telle que la pyridine.

L'estérification, la salification ou l'amidification éventuelle du radical COOH est effectuée dans les conditions classiques connues de l'homme du métier telles que, par exemple, pour l'amidification, l'utilisation d'une amine primaire ou secondaire sur un dérivé fonctionnel de l'acide par exemple un anhydride symétrique ou mixte.

Les produits objet de la présente invention possèdent d'intéressantes propriétés pharmacologiques, notamment ils se fixent sur le récepteur des androgènes et ils présentent une activité anti-androgénique.

Des tests donnés dans la partie expérimentale illustrent ces propriétés.

Ces propriétés rendent les produits de formule (I) telle que définie ci-dessus, de la présente invention utilisables comme médicaments principalement pour :
- le traitement des adénomes et des néoplasies de la prostate ainsi que l'hypertrophie bénigne de la prostate, seul ou en association avec des analogues de la LHRH. Ils peuvent également être utilisés dans le traitement de tumeurs bénignes ou malignes possédant des récepteurs aux androgènes et plus particulièrement les cancers du sein, de la peau, des ovaires, de la vessie, du système lymphatique, du rein et du foie,
- le traitement d'affections cutanées tel que l'acné, l'hyperséborrhée, l'alopécie ou l'hirsutisme. Ces produits peuvent donc être utilisés en dermatologie seuls ou en association avec des antibiotiques tels que les dérivés des acides azélaique et fusidique, l'érythromycine, ainsi que des dérivés de l'acide rétinoique ou un inhibiteur de la 5α-réductase tel que le (5α, 17β)-1,1-diméthyléthyl 3-oxo 4-aza-androst-1-ène 17-carboxamide (ou Finastéride, Merck 11ème ed.) pour le traitement de l'acné, l'alopécie ou l'hirsutisme. Ils peuvent également être associés à un produit stimulant la croissance des cheveux tel que le Minoxidil pour le traitement de l'alopécie.

Les produits de formule (I), telle que définie ci-dessus, sous forme radioactive (tritium, carbone 14, iode 125 ou fluor 18) peuvent encore être utilisés comme marqueurs spécifiques des récepteurs aux androgènes. Ils peuvent aussi être utilisés en diagnostic en imagerie médicale.

Les produits de formule (I) telle que définie ci-dessus, peuvent également être utilisés dans le domaine vétérinaire pour le traitement de troubles comportementaux tel que l'agressivité, d'affections androgénodépendantes, tel que le circum analum chez le chien et de tumeurs présentant des récepteurs aux androgènes. Ils peuvent également être utilisés pour provoquer une castration chimique chez l'animal.

L'invention a donc pour objet à titre de médicaments, les produits de formule (I) telle que définie ci-dessus, lesdits produits de formule (I) étant sous toutes les formes isomères possibles racémiques ou optiquement actives, ainsi que les sels d'addition avec les acides minéraux ou organiques ou avec les bases minérales et organiques pharmaceutiquement acceptables desdits produits de formule (I).

L'invention a également pour objet à titre de médicaments, les produits suivants :
- carbonate de 4-(3-(4-cyano-3-(trifluorométhyl)phényl)-5,5-diméthyl-2,4-dioxo-1-imidazolidinyl)butyle et de 1-méthyléthyle,
- 4-(4,4-diméthyl-2,5-dioxo-3-(4-nitrooxybutyl)-1 imidazolidinyl)-2-(trifluorométhyl)benzonitrile,
   lesdits produits de formule (I) étant sous toutes les formes isomères racémiques, énantiomères et diastéréoisomères possibles, ainsi que les sels d'addition avec les acides minéraux et organiques ou avec les bases minérales et organiques pharmaceutiquement acceptables, desdits produits de formule (I).

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule (I), telle que définie ci-dessus.

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

Les produits de formule (II) utilisés au départ de l'invention peuvent être obtenus par action du phosgène lorsque X représente un atome d'oxygène ou du thiophosgène lorsque X représente un atome de soufre sur l'amine correspondante de formule (A) : 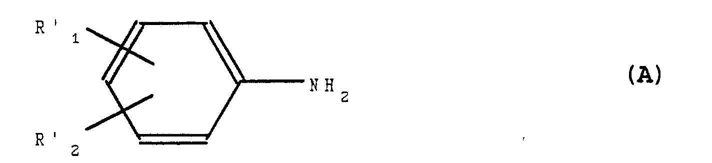 dans laquelle R'₁ et R'₂ ont les significations indiquées ci-dessus.

Un produit de ce type est notamment décrit dans le brevet français BF 2.329.276.

Des amines de formule (A) sont décrites dans le brevet européen EP 0.002.892 ou le brevet français BF 2.142.804.

Les produits de formule (III) sont connus ou peuvent être préparés à partir de la cyanhydrine correspondante selon le procédé décrit dans les publications : J. Am. Chem. Soc. (1953), 75, 4841, BEIL I 4 526 ou J. Org. Chem. 27 2901 (1962).

Les produits de formule (III) peuvent également être obtenus par action d'un produit de formule R'₃ Hal sur le 2-cyano 2-amino propane. Un exemple de préparation de ce type est décrit dans la référence :
- Jilek et Coll. Collect. Czech. Chem. Comm. 54(8) 2248 (1989).

Les produits de départ formule (VIII) telles que définies ci-dessus, sont connus et disponibles dans le commerce ou peuvent être préparés selon des méthodes connues de l'homme de métier.

Le composé de formule (VIII) peut notamment être un chloroformiate d'alkyle ou de cycloalkyle, ainsi qu'il est indiqué dans la préparation des exemples 1 à 3.

Les produits de départ de formules (VI) et (VII) peuvent être préparés notamment comme indiqué dans les demandes de brevets EP 0494819 ou EP 0580459.

L'invention a également pour objet, à titre de produits industriels et notamment à titre de produits industriels utilisables comme intermédiaires pour la préparation des produits de formule (I), telle que définie ci-dessus, les produits de formules (IV) et (V) telles que définies dans la revendication 7.

La présente invention a également pour objet l'utilisation des produits de formule (I) telle que définie ci-dessus, pour la préparation de compositions pharmaceutiques destinées au traitement des adénomes et des néoplasies de la prostate ainsi que de l'hypertrophie bénigne de la prostate, seul ou en association avec des analogues de la LHRH, au traitement d'affections cutanées tel que l'acné, l'hyperséborrhée, l'alopécie ou l'hirsutisme ou en diagnostic en imagerie médicale.

La présente invention a particulièrement pour objet l'utilisation des produits de formule (I) telle que définie ci-dessus, dans laquelle R₃ représente un radical substitué par le radical nitrooxy, pour la préparation de compositions pharmaceutiques destinées au traitement d'affections cutanées telle que l'acnée, l'alopécie ou l'hirsutisme.

Les exemples suivants illustrent l'invention.

### EXEMPLE 1 : carbonate de 4-(3-(4-cyano-3-(trifluorométhyl)-phényl)-5,5-diméthyl-2,4-dioxo-1-imidazolidinyl)butyle et de 1-méthyléthyle

on introduit 0,369 g de 4-(4,4-diméthyl-2,5-dioxo-3-(4-hydroxybutyl)-1-imidazolidinyl)-2-(trifluorométhyl)-benzonitrile obtenu à l'exemple 1 de EP 0580459, 24 ml de diméthylaminopyridine, en solution dans 3,5 mg de pyridine séchée sur potasse, refroidit à 0°C et ajoute 2 ml d'une solution toluénique de chloroformiate d'isopropyle 1 M. Après 2 h 15 d'agitation, on verse sur 30 g (eau + glace) et extrait à l'éther. Les phases éthérées sont réunies, lavées avec une solution saturée de chlorure de sodium et séchées. On reprend l'huile obtenue avec du toluène et évapore à sec sous pression réduite. Après chromatographie sur silice avec pour éluant chlorure de méthylène-acétate d'éthyle : 100-3, on obtient 0,413 g de produit attendu (cristaux blancs). F=82°C.

### ANALYSES

| | | | | |
|---|---|---|---|---|
| Microanalyse pour C₂₁H₂₄F₃N₃O₅ : 455,44 | | | | |

| | C% | H% | F% | N% |
|---|---|---|---|---|
| calculés | 55,38 | 5,31 | 12,51 | 9,23 |
| trouvés | 55,4 | 5,2 | 12,7 | 9,2 |

I.R. CHCl₃ (cm⁻¹)
Absence de OH

| | |
|---|---|
| C≡N | 2235 |
| 〉C=O | 1779-1745-1725 |
| Aromatiques | 1616-1575-1505. |

### EXEMPLE 2 : carbonate de 4-[3-[4-cyano-3-(trifluorométhyl) phényl]-5,5-diméthyl-2,4-dioxo-1-imidazolidinyl] butyle et de cyclohexyle

On procède comme à l'exemple 1, à partir de 740 mg de 4-(4,4-diméthyl-2,5-dioxo-3-(4-hydroxybutyl)-1-imidazolidinyl)-2-(trifluorométhyl)-benzonitrile obtenu à l'exemple 1 de EP 0580459, 48 mg de 4-diméthylaminopyridine et 7 ml de pyridine, refroidit à 10°C ± 2°C et ajoute 650 mg chloroformiate de cyclohexyle. On procède comme à l'exemple 1 et après chromatographie sur silice avec pour éluant chlorure de méthylène-acétate d'éthyle : 97-3, on obtient 948 mg de produit attendu. F = 121-122°C.

### Analyses :

| Microanalyse pour C₂₄H₂₈F₃N₃O₅ : 495,5 | | | | |
|---|---|---|---|---|
| | C% | H% | F% | N% |
| calculés | 58,18 | 5,70 | 11,50 | 8,48 |
| trouvés | 58,2 | 5,8 | 11,3 | 8,4 |

IR (CHCl₃) cm⁻¹

| | |
|---|---|
| C≡N | 2235 |
| >=O | 1779 - 1725 |
| Aromatiques | 1614 - 1579 - 1552 - 1505 |

### EXEMPLE 3 : carbonate de 4-[3-[4-cyano-3-(trifluorométhyl) phényl]-5,5-diméthyl-2,4-dioxo-1-imidazolidinyl] butyle et de cyclopentyle

On procède comme à l'exemple 2, à partir de 740 mg de 4-(4,4-diméthyl-2,5-dioxo-3-(4-hydroxybutyl)-1-imidazolidinyl)-2-(trifluorométhyl)-benzonitrile obtenu à l'exemple 1 de EP 0580459, 48 mg de 4-diméthylaminopyridine et 7 ml de pyridine, refroidit au bain de glace et ajoute 600 mg de chloroformiate de cyclopentyle. En procédant comme à l'exemple 2, on obtient 904 mg de produit attendu (cristaux blancs). F = 81-82°C.

### Analyses :

| Microanalyse pour C₂₃H₂₆F₃N₃O₅ : 481,47 | | | | |
|---|---|---|---|---|
| | C% | H% | %F | N% |
| calculés | 57,37 | 5,44 | 11,84 | 8,73 |
| trouvés | 57,5 | 5,4 | 11,7 | 8,7 |

IR (CHCl₃) cm⁻¹

| | |
|---|---|
| C≡N | 2238 |
| >=O | 1779 - 1725 |
| Aromatiques | 1612 - 1575 - 1505 |

### EXEMPLE 4 : 4-(4,4-diméthyl-2,5-dioxo-3-(4-nitrooxybutyl)-1 imidazolidinyl)-2-(trifluorométhyl)-benzonitrile

### STADE 1 : 4-(4,4-diméthyl-2,5-dioxo-3-(4-iodobutyl)-1-imidazolidinyl)-2-(trifluorométhyl)-benzonitrile

On introduit 787 mg de triphényl phosphine, 204 mg d'imidazole, 762 mg d'iode, 5 ml de chlorure de méthylène, agite la suspension 45 mn à température ambiante, puis ajoute en 3 mn environ, 1,1 g de 4-(4,4-diméthyl-2,5-dioxo-3-(4-hydroxybutyl)-1-imidazolidinyl)-2-(trifluorométhyl)-benzonitrile obtenu à l'exemple 1 de EP 0580459 dans 7 ml de chlorure de méthylène. On rince avec 1 ml de chlorure de méthylène et agite 4 heures à température ambiante.

On essore l'insoluble, rince avec du chlorure de méthylène, lave la phase organique avec une solution aqueuse saturée de thiosulfate de sodium puis à l'eau salée et sèche. On chromatographie sur silice avec pour éluant chlorure de méthylène-acétate d'éthyle : 97-3. Après cristallisation dans l'éther, on obtient 1,26 g de produit attendu (cristaux blancs). F = 87-88°C.

### Analyses physiques :

| Microanalyses pour C₁₇H₁₇F₃IN₃O₂ : 479,24 | | | | | |
|---|---|---|---|---|---|
| | C% | H% | FM | I% | N% |
| calculés | 42,61 | 3,57 | 11,89 | 26,48 | 8,77 |
| trouvés | 42,7 | 3,3 | 11,9 | 26,2 | 8,5 |

IR (CHCl₃) cm⁻¹

| | |
|---|---|
| C≡N | 2238 |
| C=O | 1779 - 1725 |
| Aromatiques | 1615 - 1579 - 1505 |

UV (EtOH)

| | |
|---|---|
| Max 261 nm | ε = 14900 |
| infl 286 nm | ε = 3500 |

### STADE 2 : 4-(4,4-diméthyl-2,5-dioxo-3-(4-nitrooxybutyl)-1 imidazolidinyl)-2-(trifluorométhyl)-benzonitrile

On introduit 1 g du produit obtenu au stade 1 ci-dessus, dans 10 ml d'acétonitrile et ajoute à la solution obtenue, 426 mg de nitrate d'argent. On laisse agir 16 heures à température ambiante, essore l'insoluble, rince 2 fois au chlorure de méthylène et évapore. Après purification par chromatographie sur silice avec pour éluant chlorure de méthylène-acétate d'éthyle : 99-1, on obtient 845 mg de produit attendu (cristaux blancs). F = 74-75°C.

### Analyses physiques :

| Microanalyses pour C₁₇H₁₇F₃N₄O₅ : 414,34 | | | | |
|---|---|---|---|---|
| | C% | H% | F% | N% |
| calculés | 49,28 | 4,13 | 13,75 | 13,52 |
| % trouvés | 49,2 | 4,0 | 13,7 | 13,5 |

IR (CHCl₃) cm⁻¹

| | |
|---|---|
| C=N | 2238 |
| >=O | 1780 - 1725 |
| ONO₂ | 1636 |
| Aromatiques | 1616 - 1575 - 1505 |

UV (EtOH)

| | |
|---|---|
| Max 261 nm | ε = 15500 |
| infl 286 nm | ε = 3500 |

### EXEMPLE 5 :

On a préparé des comprimés ayant la composition suivante:
- Produit de l'exemple 1 100 mg
- Excipient q.s. pour un comprimé terminé à 300 mg (Détail de l'excipient : lactose, amidon, talc, stéarate de magnésium).

### ETUDE PHARMACOLOGIOUE DES PRODUITS DE L'INVENTION

### 1) Etude de l'affinité des produits de l'invention pour le récepteur androgène

Des rats mâles Sprague Dawley EOPS de 180-200 g, castrés de 24 heures, sont sacrifiés, les prostates prélevées, pesées et homogénéisées à 0°C à l'aide d'un potter verre-verre, dans une solution tamponnée (Tris 10mM, saccharose 0,25M, PMSF (phénylméthanesulfonylfluoride) 0,1mM, Molybdate de sodium 20mM, HCl pH 7,4 ; auxquels on ajoute extemporanément 2mM de DTT (DL dithiothreitol), à raison de 1 g de tissu pour 8 ml de tampon.

L'homogénat est ensuite ultracentrifugé à 0°C, 30 minutes à 209 000 g. Des aliquotes du surnageant obtenu (=cytosol), sont incubées 30 minutes et 24 heures à 0°C, avec une concentration constante (T) de Testostérone tritiée et en présence de concentrations croissantes (0 à 2500.10⁻⁹M), soit de testostérone froide, soit des produits à tester. La concentration de Testostérone tritiée liée (B) est ensuite mesurée dans chaque incubat par la méthode d'adsorption au charbon-dextran.

### Calcul de l'affinité relative de liaison (ARL).

On trace les 2 courbes suivantes : le pourcentage de l'hormone tritiée liée B/T en fonction du logarithme de la concentration de l'hormone de référence froide et B/T en fonction du logarithme de la concentration du produit froid testé. On détermine la droite d'équation
I₅₀= (B/Tmax + B/Tmin)/2.
B/T max= % de l'hormone tritiée liée pour une incubation de cette hormone tritiée à la concentration (T).
B/T min= % de l'hormone tritiée liée pour une incubation de cette hormone tritiée à la concentration (T) en présence d'un grand excès d'hormone froide (2500.10⁻⁹M).

Les intersections de la droite I₅₀ et des courbes, permettent d'évaluer les concentrations de l'hormone de référence froide (CH) et du produit froid testé (CX) qui inhibent de 50 % la liaison de l'hormone tritiée sur le récepteur. L'affinité relative de liaison (ARL) du produit testé est déterminé par l'équation ARL=100 (CH)/(CX). On obtient les résultats suivants exprimés en ARL.

Produit de référence (Testostérone) : 100

**TABLEAU 1**

| Produit de l'exemple | ARL : Incubation 24 heures |
|---|---|
| 1 | 19 |

### 2) Détermination de l'effet de réduction sur la glande costovertébrale du hamster

L'activité locale (topique) d'un antiandrogène se détermine par la réduction qu'il entraîne de la surface de la glande costovertébrale du hamster (ci-après G.C.V.), organe androgénodépendant se situant sur les flancs de l'animal.

Les animaux sont des hamsters mâles pesant environ 140 g, âgés de 14 semaines provenant de l'élevage Charles River (USA), ils sont maintenus en photopériode longue (16 h de lumière, 8 h d'obscurité). Les animaux sont traités tous les jours, exception faite du week-end, pendant 3 semaines (14 administrations). Le produit à tester est appliqué, par voie topique, sur la G.C.V. droite, la gauche servant de témoin, la surface de la glande étant préalablement rasée. Les animaux sont sacrifiés par saignée carotidienne 24 h après le dernier traitement. Les G.C.V. sont prélevées, mesurées et pesées. L'activité locale d'un produit est déterminée par le % de diminution de la surface de la G.C.V. qu'il induit comparativement au 1er jour de l'expérience et comparativement aux animaux traités par le solvant seul.

**TABLEAU 2**

| Produit de l'exemple | % réduction GCV à 3 *µ*g/jour |
|---|---|
| 1 | -32 |

### 3) Détermination de l'effet de réduction du poids de la prostate chez le rat mâle entier

L'activité systémique d'un antiandrogène est déterminée par la réduction du poids de la prostate qu'il entraîne chez un animal entier.

Les animaux utilisés sont des rats mâles de souche Sprague Dawley pesant environ 200 g, âgés de 7 semaines, provenant de l'élevage Iffa Credo (France). L'expérience se déroule sur deux semaines, exception faite du week-end.

Le produit peut être administré par voie orale, sous-cutanée ou percutanée.

Les solvants utilisés sont alors :
par voie orale : solution aqueuse 0,5 % de méthylcellulose sous un volume de 5 ml/kg,
par voie sous-cutanée : huile de germe de mais 10 % d'éthanol sous un volume de 0,2 ml/kg,
et par voie percutanée : éthanol sous un volume de 50 *µ*l sur la peau préalablement rasée.

Le traitement s'effectue du jour 0 au jour 4 puis (après le week-end) du jour 7 au jour 10. Les animaux sont sacrifiés le lendemain du dernier traitement par saignée carotidienne, les prostates sont prélevées et fixées dans de l'eau déminéralisée contenant 10 % de formol pendant 72 h. Elles sont ensuite disséquées et pesées. Le sang est prélevé afin de déterminer, par dosage radioimmunologique, le taux de testostérone sérique. L'activité antiandrogénique du produit est exprimée en % de réduction du poids des prostates et en % de variation des taux de testostérone comparativement aux animaux traités par le solvant seul.

**TABLEAU 3**

| Produit de l'exemple | % réduction du poids de prostate à 3 mg/kg P.O. |
|---|---|
| 1 | -5 |

## Revendications

1. Produits de formule (I) : 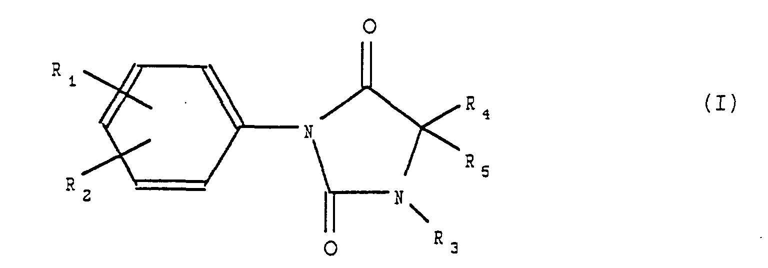 dans laquelle :
R₁ et R₂ représentent un radical cyano et un radical trifluorométhyle,
R₃ représente un radical alkyle, alkényle ou alkynyle, linéaires ou ramifiés, renfermant au plus 4 atomes de carbone substitué par un radical choisi parmi les radicaux nitrooxy et 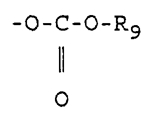 dans lequel R₉ représente, un radical alkyle, linéaire ou ramifié, renfermant au plus 4 atomes de carbone ou un radical cycloalkyle renfermant 5 ou 6 chaînons,
R₄ et R₅ soit, identiques ou différents, représentent un radical méthyle éventuellement substitué par un atome de fluor,
soit forment avec l'atome de carbone auquel ils sont attachés un radical cyclohexyle
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

2. Produits de formule (I) telle que définie à la revendication 1, dont les noms suivent :
- carbonate de 4-(3-(4-cyano-3-(trifluorométhyl)phényl)-5,5-diméthyl-2,4-dioxo-1-imidazolidinyl)butyle et de 1-méthyléthyle,
- 4-(4,4-diméthyl-2,5-dioxo-3-(4-nitrooxybutyl)-1 imidazolidinyl)-2-(trifluorométhyl)benzonitrile,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I).

3. Procédé de préparation des produits de formule (I), telle que définie à la revendication 1, **caractérisé en ce que** :
soit l'on fait agir en présence d'une base tertiaire un produit de formule (II) : 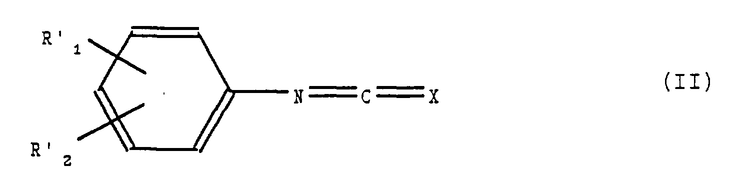 dans laquelle R'₁ et R'₂ ont les significations indiquées à la revendication 1 respectivement pour R₁ et R₂, dans lesquelles les éventuelles fonctions réactives sont éventuellement protégées et X représente un atome d'oxygène ou de soufre , avec un produit de formule (III) : 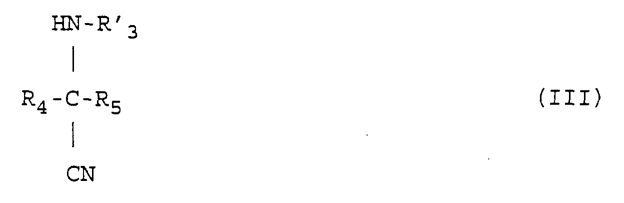 dans laquelle R₄ et R₅ ont les significations indiquées à la revendication 1 et R'₃ a la signification indiquée à la revendication 1 pour R₃, dans lesquelles les éventuelles fonctions réactives sont éventuellement protégées, pour obtenir un produit de formule (IV) : 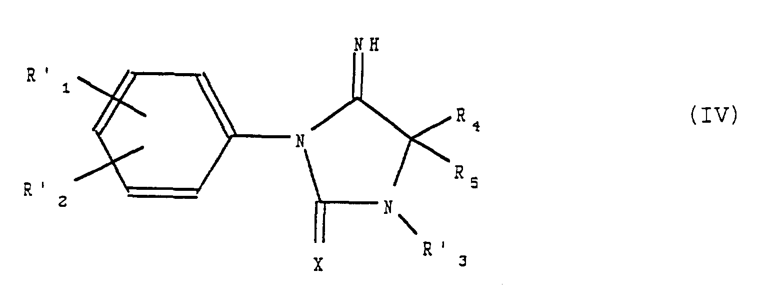 dans laquelle X, R'₁, R'₂, R'₃, R₄ et R₅ ont les significations indiquées ci-dessus,
que l'on transforme en un produit de formule (V) : 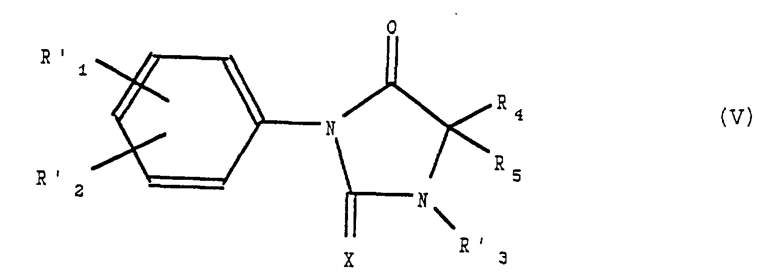 dans laquelle X, R'₁, R'₂, R'₃, R₄ et R₅ ont les significations indiquées ci-dessus,
soit l'on fait agir un produit de formule (VI) : 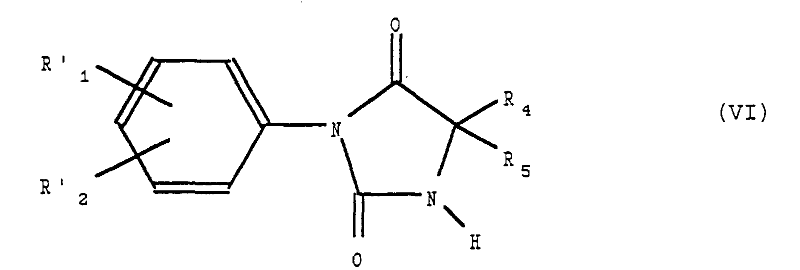 dans laquelle R'₁, R'₂, R₄ et R₅ ont les significations indiquées ci-dessus, avec un réactif de formule Hal-R'₃ dans laquelle R'₃ a la signification indiquée ci-dessus, et Hal représente un atome d'halogène pour obtenir les produits correspondants de formule (V) telle que définie ci-dessus, dans laquelle X représente un atome d'oxygène,
soit l'on fait agir sur un produit de formule (VII) : 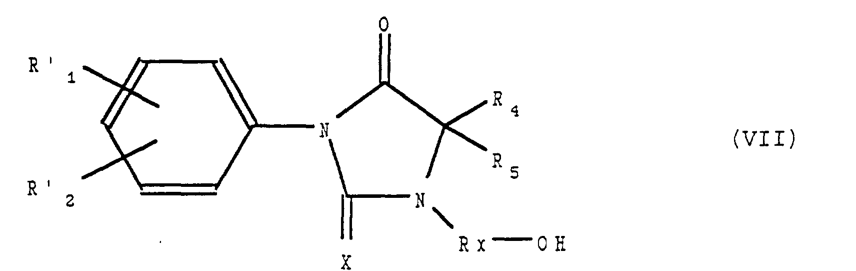 dans laquelle R'₁, R'₂, R₄, R₅ et X ont les significations indiquées ci-dessus,
et Rx représente un radical alkyle, alkényle ou alkynyle, linéaires ou ramifiés, renfermant au plus 4 atomes de carbone, que l'on soumet :
**ou bien** à l'action d'un composé de formule (VIII) : 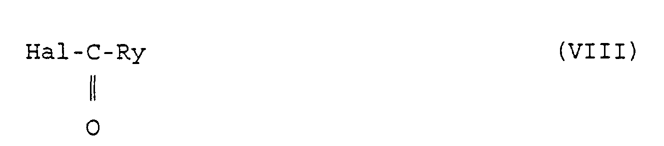 dans laquelle Hal représente un atome d'halogène et Ry représente un radical choisi parmi les radicaux alcoxy, cycloalcoxy, tels que définis ci-dessus, pour obtenir un produit de formule (IX) : 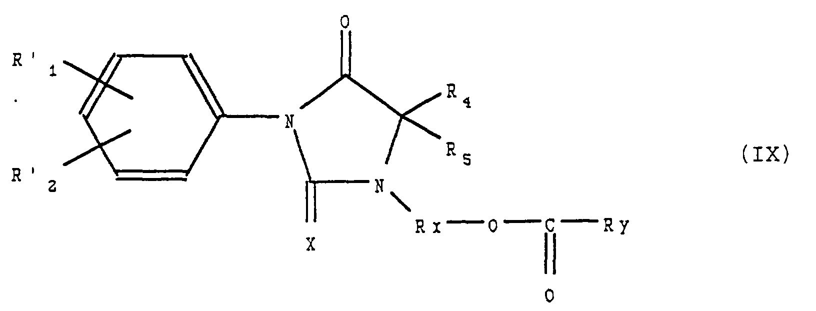 dans laquelle R'₁, R'₂, R₄, R₅, X, Rx et Ry ont les significations indiquées ci-dessus,
**ou bien** à une réaction d'halogénation pour obtenir un produit de formule (X) : 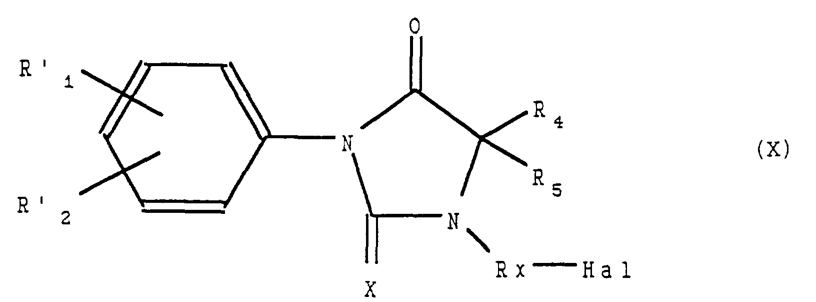 dans laquelle R'₁, R'₂, R₄, R₅, X, Rx et Hal ont les significations indiquées ci-dessus, que l'on soumet à l'action de nitrate d'argent pour obtenir le produit de formule (XI) : 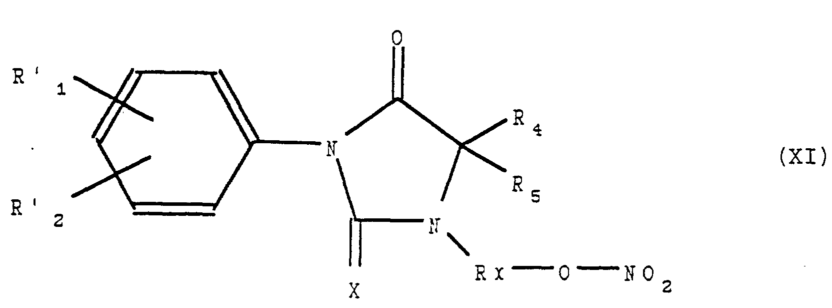 dans laquelle R'₁, R'₂, R₄, R₅, X, Rx et Hal ont les significations indiquées ci-dessus, produits de formules (IV), (V), (IX) et (XI) que, si nécessaire ou si désiré, pour obtenir des produits de formule (I) telle que définie à la revendication 1, l'on peut soumettre à l'une quelconque ou plusieurs des réactions suivantes, dans un ordre quelconque :
a) le cas échéant transformation du groupement >C=S que peut représenter >C=X en groupement >C=O,
b) libération du radical OH que peut porter R'₃,
c) réaction d'estérification ou éthérification du radical OH que peut porter R₃,
d) réaction d'élimination des éventuels groupements protecteurs,
e) le cas échéant action d'un agent d'estérification, d'amidification ou de salification,
f) réaction de dédoublement des formes racémiques,
lesdits produits de formule (I) ainsi obtenus étant sous toutes les formes isomères possibles racémiques, énantiomères et diastéréoisomères.

4. A titre de médicaments, les produits de formule (I) telles que définies aux revendications 1 et 2, lesdits produits de formule (I) étant sous toutes les formes isomères racémiques, énantiomères et diastéréoisomères possibles, ainsi que les sels d'addition avec les acides minéraux et organiques ou avec les bases minérales et organiques pharmaceutiquement acceptables, desdits produits de formule (I).

5. A titre de médicaments, les produits suivants tels que définis aux revendications 1 et 2 :
- carbonate de 4-(3-(4-cyano-3-(trifluorométhyl)phényl)-5,5-diméthyl-2,4-dioxo-1-imidazolidinyl)butyle et de 1-méthyl-éthyle,
- 4-(4,4-diméthyl-2,5-dioxo-3-(4-nitrooxybutyl)-1 imidazolidinyl)-2-(trifluorométhyl)benzonitrile,
lesdits produits de formule (I) étant sous toutes les formes isomères racémiques, énantiomères et diastéréoisomères possibles, ainsi que les sels d'addition avec les acides minéraux et organiques ou avec les bases minérales et organiques pharmaceutiquement acceptables, desdits produits de formule (I).

6. Les compositions pharmaceutiques contenant, à titre de principe actif, l'un au moins des médicaments tels que définis à l'une quelconque des revendications 4 et 5.

7. A titre de produits industriels, les produits de formules (IV) et (V) 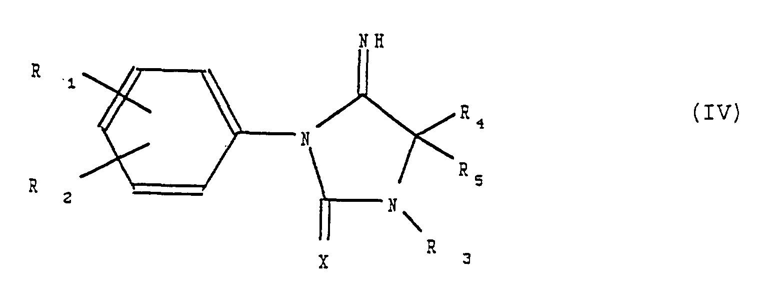 dans laquelle R ₁, R ₂' R ₃, R₄ et R₅ ont les significations indiquées dans la revendication 1 et X représente un atome d'oxygène ou de soufre 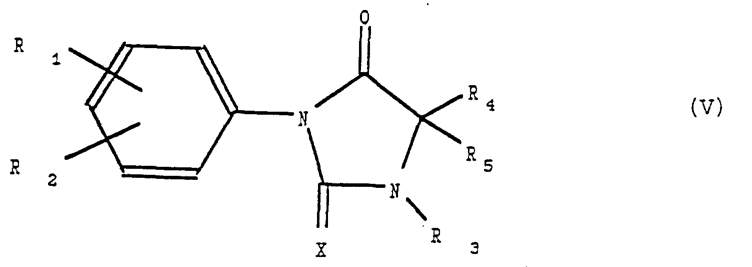 dans laquelle R ₁, R ₂, R ₃, R₄ et R₅ ont les significations indiquées dans la revendication 1 et X représente un atome de soufre

8. Utilisation des produits de formule (I) telle que définie aux revendications 1 et 2, pour la préparation de compositions pharmaceutiques destinées au traitement des adénomes et des néoplasies de la prostate ainsi que de l'hypertrophie bénigne de la prostate, seul ou en association avec des analogues de la LHRH, au traitement d'affections cutanées telles que l'acné, l'hyperséborrhée, l'alopécie ou l'hirsutisme ou en diagnostic en imagerie médicale.

9. Utilisation des produits de formule (I) telle que définie aux revendications 1 et 2, dans laquelle R₃ représente un radical substitué par le radical nitrooxo, pour la préparation de compositions pharmaceutiques destinées au traitement d'affections cutanées telle l'acnée, l'alopécie ou l'hirsutisme.

## Patentansprüche

1. Produkte der Formel (I) 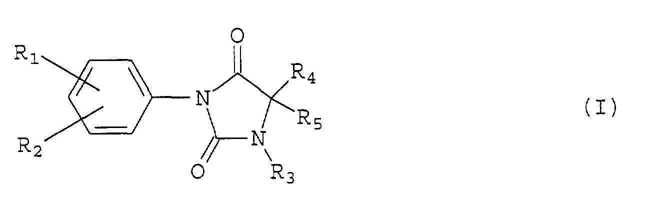 in der
R₁ und R₂ einen Rest Cyano und einen Rest Trifluormethyl darstellen,
R₃ einen geraden oder verzweigten Rest Alkyl, Alkenyl oder Alkinyl mit höchstens 4 Kohlenstoffatomen bedeutet, substituiert durch einen Rest, ausgewählt unter den Resten Nitrooxy und 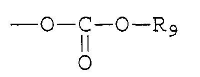 worin R₉ einen geraden oder verzweigten Rest Alkyl mit höchstens 4 Kohlenstoffatomen oder einen Rest Cycloalkyl mit 5 oder 6 Ringgliedern darstellt, entweder R₄ und R₅, gleich oder verschieden, einen Rest Methyl bedeuten, gegebenenfalls substituiert durch ein Fluoratom, oder zusammen mit dem Kohlenstoff, an den sie gebunden sind, einen Rest Cyclohexyl bilden,
wobei die genannten Produkte der Formel (I) in allen möglichen racemischen isomeren Formen, Enantiomeren und Diastereoisomeren vorliegen können, sowie die Additionssalze der genannten Produkte der Formel (I) mit Mineralsäuren und organischen Säuren oder mit Mineralbasen und organischen Basen.

2. Produkte der Formel (I) wie in Anspruch 1 definiert, mit den folgenden Namen:
- Carbonat von 4-{3-[4-Cyano-3-(trifluormethyl)-phenyl]-5,5-dimethyl-2,4-dioxo-1-imidazolidinyl}-butyl und von 1-Methylethyl,
- 4-[4,4-Dimethyl-2,5-dioxo-3-(4-nitrooxybutyl)-1-imidazolidinyl]-2-(trifluormethyl)-benzonitril, wobei die genannten Produkte der Formel (I) in allen möglichen racemischen isomeren Formen, Enantiomeren und Diastereoisomeren vorliegen können, sowie die Additionssalze der genannten Produkte der Formel (I) mit Mineralsäuren und organischen Säuren oder mit Mineralbasen und organischen Basen.

3. Verfahren zur Herstellung der Produkte der Formel (I) wie in Anspruch 1 definiert, **dadurch gekennzeichnet, daß** man entweder ein Produkt der Formel (II) 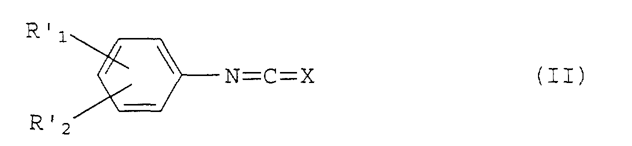 in der R'₁ und R'₂ die in Anspruch 1 für R₁ und R₂ angegebenen Bedeutungen besitzen, in denen die eventuellen reaktiven Funktionen gegebenenfalls geschützt sind und X ein Sauerstoffatom oder ein Schwefelatom darstellt, in Anwesenheit einer tertiären Base mit einem Produkt der Formel (III) 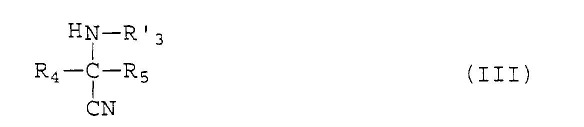 in der R₄ und R₅ die in Anspruch 1 angegebenen Bedeutungen besitzen und R'₃ die in Anspruch 1 für R₃ angegebene Bedeutung besitzt, in denen die eventuellen reaktiven Funktionen gegebenenfalls geschützt sind, zur Reaktion bringt, um ein Produkt der Formel (IV) 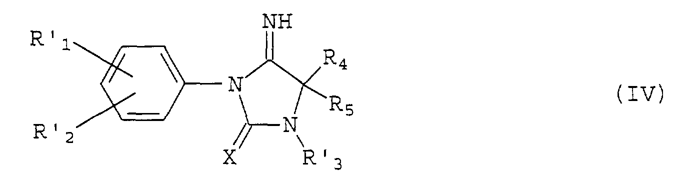 zu erhalten, in der X, R'₁, R'₂, R'₃, R₄ und R₅ die oben angegebenen Bedeutungen besitzen, das man in ein Produkt der Formel (V) 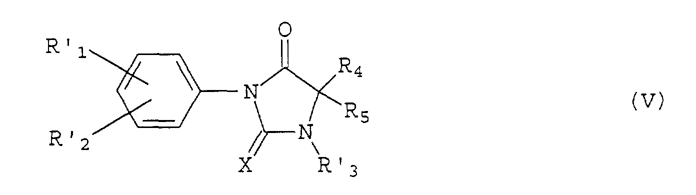 überführt, in der X, R'₁, R'₂, R'₃, R₄ und R₅ die oben angegebenen Bedeutungen besitzen,
oder ein Produkt der Formel (VI) 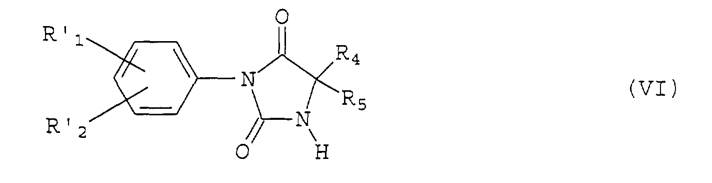 in der R'₁, R'₂, R₄ und R₅ die oben angegebenen Bedeutungen besitzen, mit einem Reaktanden der Formel Hal-R'₃, worin R'₃ die oben angegebene Bedeutung besitzt und Hal ein Halogenatom ist, zur Reaktion bringt, um die wie oben definierten Produkte der Formel (V) zu erhalten, worin X ein Sauerstoffatom ist,
oder ein Produkt der Formel (VII) 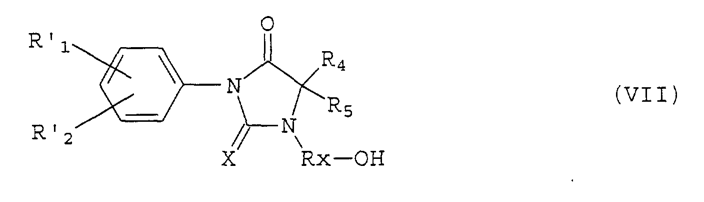 in der R'₁, R'₂, R₄, R₅ und X die oben angegebenen Bedeutungen besitzen und Rx einen geraden oder verzweigten Rest Alkyl, Alkenyl oder Alkinyl mit höchstens 4 Kohlenstoffatomen darstellt, zur Reaktion bringt, das man unterzieht:
entweder der Einwirkung einer Verbindung der Formel (VIII) 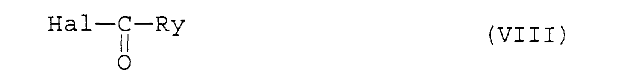
in der Hal ein Halogenatom ist und Ry einen Rest darstellt, ausgewählt unter den Resten Alkoxy, Cycloalkoxy, wie oben definiert, um ein Produkt der Formel (IX) 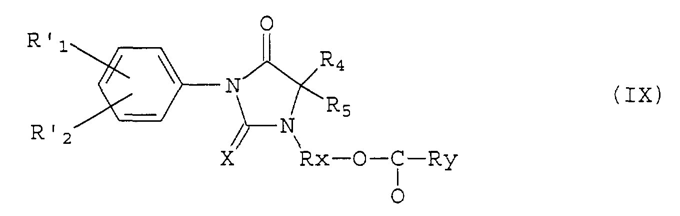
zu erhalten, in der R'₁, R'₂, R₄, R₅, X, Rx und Ry die oben angegebenen Bedeutungen besitzen,
oder einer Reaktion zur Halogenierung, um ein Produkt der Formel (X) 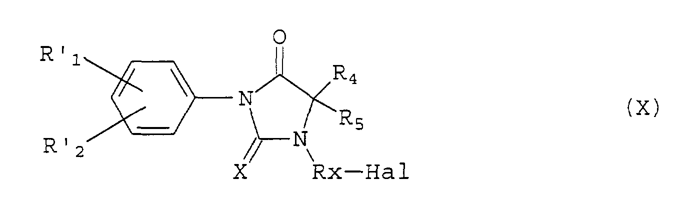
zu erhalten, in der R'₁, R'₂, R₄, R₅, X, Rx und Hal die oben angegebenen Bedeutungen besitzen,
das man der Einwirkung von Silbernitrat unterwirft, um das Produkt der Formel (XI) 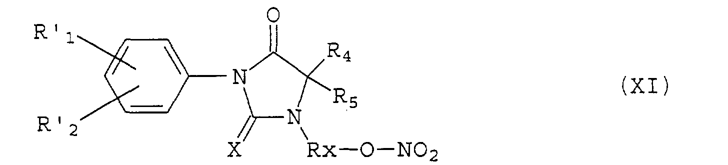
zu erhalten, in der R'₁, R'₂, R₄, R₅, X, Rx und Hal die oben angegebenen Bedeutungen besitzen,
wobei man die Produkte der Formeln (IV), (V), (IX) und (X), wenn erforderlich oder wenn erwünscht, zur Herstellung der wie in Anspruch 1 definierten Produkte der Formel (I) irgendeiner oder mehreren der folgenden Reaktionen in irgendeiner Reihenfolge unterziehen kann:
a) gegebenenfalls Umwandlung der Gruppe >C=S, die von >C=X dargestellt werden kann, in die Gruppe >C=O,
b) Freisetzung des Restes OH, den R'₃ tragen kann,
c) Reaktion zur Veresterung oder Veretherung des Restes OH, den R₃ tragen kann,
d) Reaktion zur Entfernung der eventuellen Schutzgruppen,
e) gegebenenfalls Einwirkung eines Mittels zur Veresterung, Amidierung oder Salzbildung,
f) Reaktion zur Spaltung der racemischen Formen,
wobei die auf diese Weise erhaltenen Produkte der Formel (I) in. allen möglichen racemischen isomeren Formen, Enantiomeren und Diastereoisomeren vorliegen können.

4. Als Arzneimittel die Produkte der Formel (I) wie in den Ansprüchen 1 und 2 definiert, wobei die genannten Produkte der Formel (I) in allen möglichen racemischen isomeren Formen, Enantiomeren und Diastereoisomeren vorliegen können, sowie die Additionssalze der genannten Produkte der Formel (I) mit pharmazeutisch akzeptablen Mineralsäuren und organischen Säuren oder mit Mineralbasen und organischen Basen.

5. Als Arzneimittel die folgenden Produkte wie in Anspruch 1 und 2 definiert:
- Carbonat von 4-{3-[4-Cyano-3-(trifluormethyl)-phenyl]-5,5-dimethyl-2,4-dioxo-1-imidazolidinyl}-butyl und von 1-Methylethyl,
- 4-[4,4-Dimethyl-2,5-dioxo-3-(4-nitrooxybutyl)-1-imidazolidinyl]-2-(trifluormethyl)-benzonitril, wobei die genannten Produkte der Formel (I) in allen möglichen racemischen isomeren Formen, Enantiomeren und Diastereoisomeren vorliegen können, sowie die Additionssalze der genannten Produkte der Formel (I) mit pharmazeutisch akzeptablen Mineralsäuren und organischen Säuren oder mit Mineralbasen und organischen Basen.

6. Pharmazeutische Zusammensetzungen, die als Wirkstoff mindestens eines der wie in irgendeinem der Ansprüche 4 und 5 definierten Arzneimittel enthalten.

7. Als industrielle Produkte die Produkte der Formeln (IV) und (V) 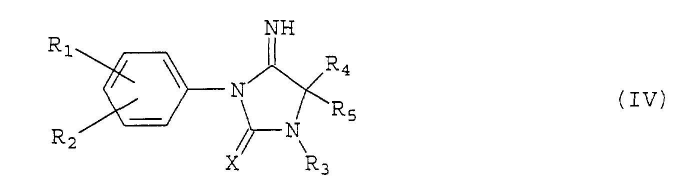 in der R₁, R₂, R₃, R₄ und R₅ die in Anspruch 1 angegebenen Bedeutungen besitzen und X ein Sauerstoffatom oder ein Schwefelatom ist, 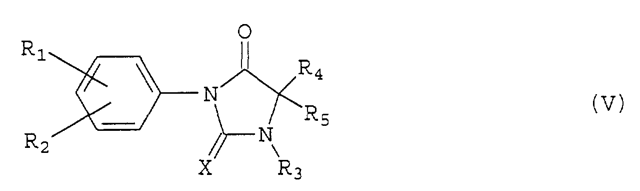 in der R₁, R₂, R₃, R₄ und R₅ die in Anspruch 1 angegebenen Bedeutungen besitzen und X ein Schwefelatom ist.

8. Verwendung der Produkte der Formel (I) wie in Anspruch 1 und 2 definiert zur Herstellung von pharmazeutischen Zusammensetzungen, vorgesehen für die Behandlung von Adenomen und Neoplasien der Prostata sowie der benignen Hypertrophie der Prostata, allein oder in Assoziation mit Analogen von LHRH, für die Behandlung von kutanen Erkrankungen wie Akne, Hyperseborrhoe, Alopezie oder Hirsutismus oder in der Diagnostik mit Hilfe der medizinischen Bilddarstellung.

9. Verwendung der Produkte der Formel (I) wie in Anspruch 1 und 2 definiert, worin R₃ einen durch den Rest Nitrooxo substituierten Rest darstellt, zur Herstellung von pharmazeutischen Zusammensetzungen, vorgesehen für die Behandlung von kutanen Erkrankungen wie Akne, Alopezie oder Hirsutismus.

## Claims

1. Products of formula (I): 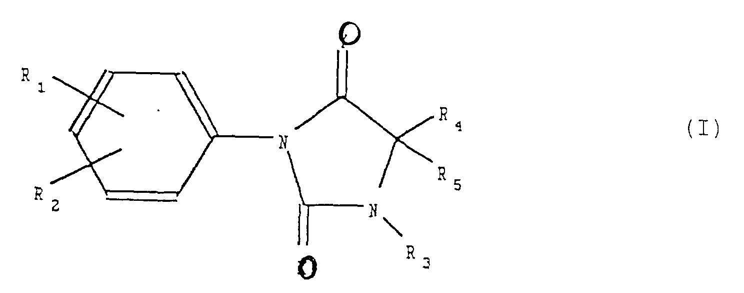 in which:
R₁ and R₂ represent a cyano radical and a trifluoromethyl radical,
R₃ represents a linear or branched alkyl, alkenyl or alkynyl radical, containing at least 4 carbon atoms substituted by a radical chosen from the nitrooxy and 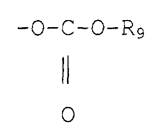 radicals in which R₉ represents, a linear or branched alkyl radical containing at most 4 carbon atoms or a cycloalkyl radical containing 5 or 6 members,
R₄ and R₅ either, identical or different, represent a methyl radical optionally substituted by a fluorine atom, or form with the carbon atom to which they are attached a cyclohexyl radical
said products of formula (I) being in all possible racemic, enantiomeric and diastereoisomeric isomer forms, as well as the addition salts with mineral and organic acids or with mineral and organic bases of said products of formula (I).

2. Products of formula (I) as defined in claim 1, the names of which follow:
- carbonate of 4-(3-(4-cyano-3-(trifluoromethyl)phenyl)-5,5-dimethyl-2,4-dioxo-1-imidazolidinyl)butyl and of 1-methylethyl,
- 4-(4,4-dimethyl-2,5-dioxo-3-(4-nitrooxybutyl)-1 imidazolidinyl)-2-(trifluoromethyl)benzonitrile,
said products of formula (I) being in all possible racemic, enantiomeric and diastereoisomeric isomer forms, as well as the addition salts with mineral and organic acids or with mineral and organic bases of said products of formula (I).

3. Process for the preparation of the products of formula (I), as defined in claim 1, **characterized in that**:
either a product of formula (II): 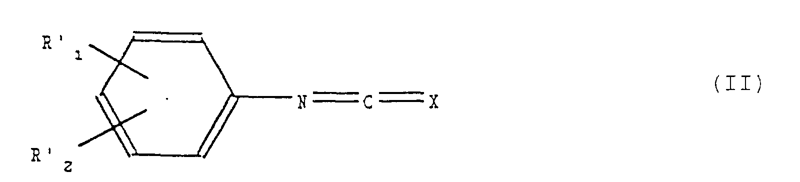 in which R'₁ and R'₂ have the meanings indicated in claim 1 for R₁ and R₂ respectively, in which the optional reactive functions are optionally protected and X represents an oxygen or sulphur atom, is reacted with a product of formula (III)
in the presence of a tertiary base: 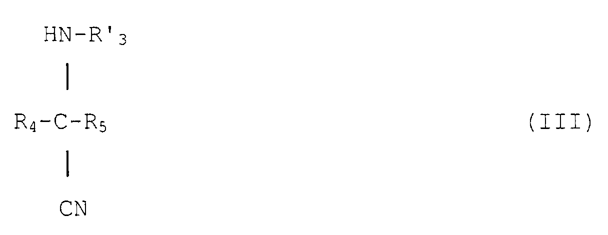 in which R₄ and R₅ have the meanings indicated in claim 1 and R'₃ has the meaning indicated in claim 1 for R₃, in which the optional reactive functions are optionally protected, in order to obtain a product of formula (IV): 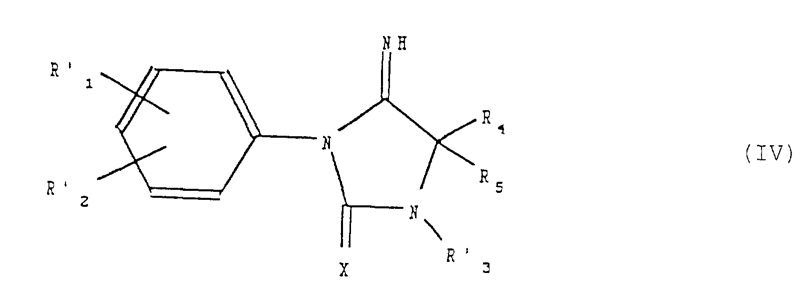 in which X, R'₁, R'₂, R'₃, R₄ and R₅ have the meanings indicated above,
which is converted to a product of formula (V): 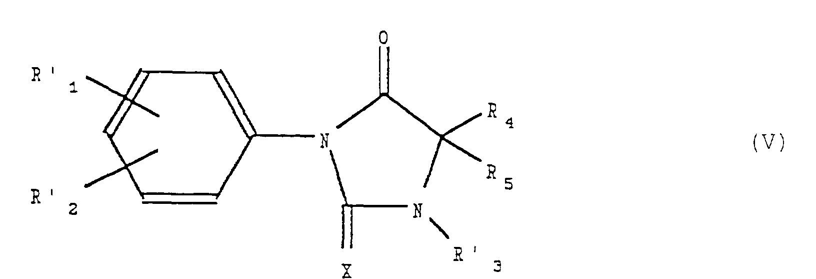 in which X, R'₁, R'₂, R'₃, R₄ and R₅ have the meanings indicated above,
or a product of formula (VI): 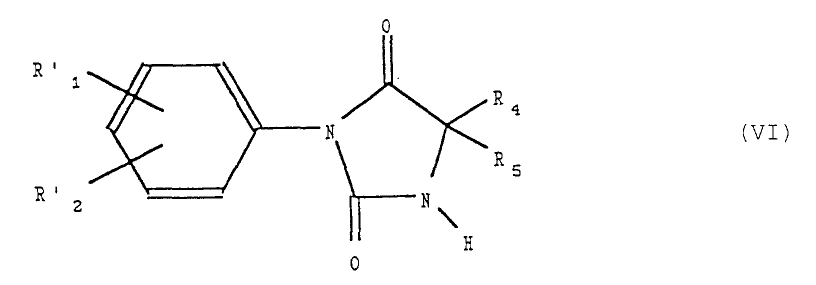 in which R'₁, R'₂, R₄ and R₅ have the meanings indicated above, is reacted with a reagent of formula Hal-R'₃ in which R'₃ has the meaning indicated above, and Hal represents a halogen atom in order to obtain the corresponding products of formula (V) as defined above, in which X represents an oxygen atom, or a product of formula (VII): 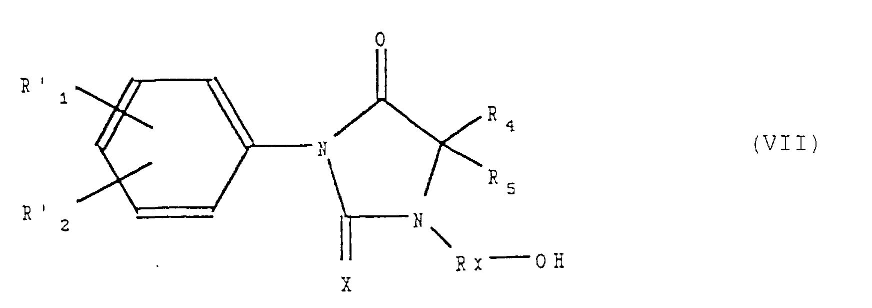 in which R'₁, R'₂, R₄, R₅ and X have the meanings indicated above,
and Rx represents a linear or branched alkyl, alkenyl or alkynyl radical, containing at most 4 carbon atoms, which is subjected:
**either** to the action of a compound of formula (VIII): 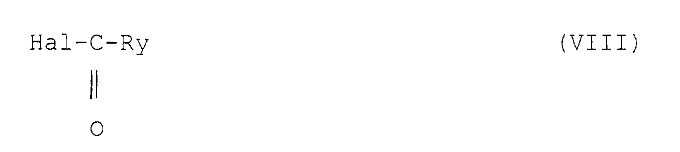 in which Hal represents a halogen atom and Ry represents a radical chosen from the alkoxy, cycloalkoxy radicals, as defined above, in order to obtain a product of formula (IX): 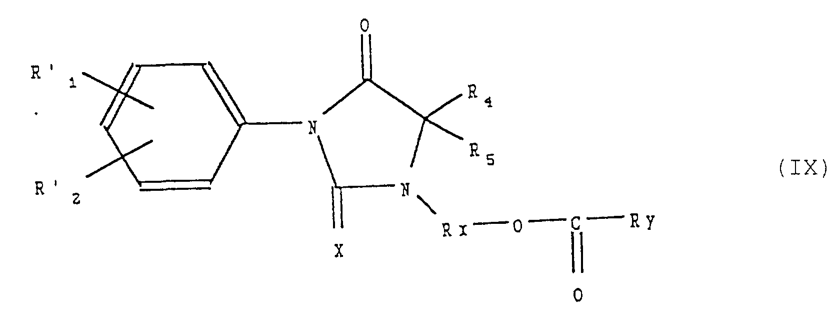 in which R'₁, R'₂, R₄, R₅, X, Rx and Ry have the meanings indicated above,
**or** to a halogenation reaction in order to obtain a product of formula (X): 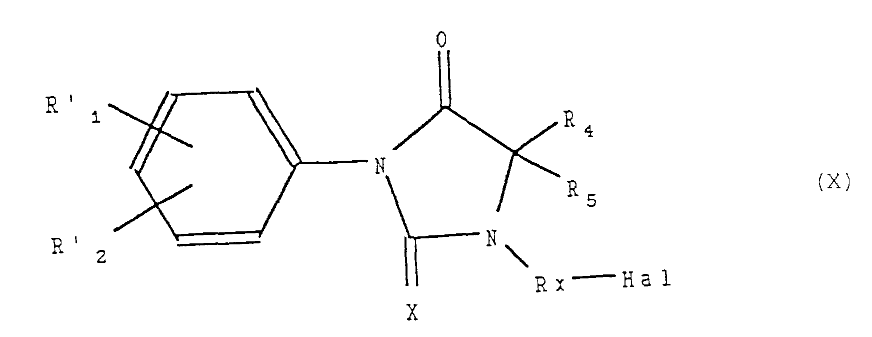 in which R'₁, R'₂, R₄, R₅, X, Rx and Hal have the meanings indicated above,
which is subjected to the action of silver nitrate in order to obtain the product of formula (XI): 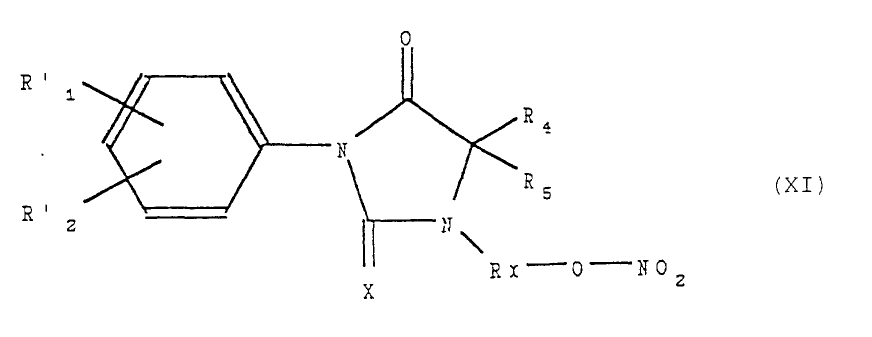 in which R'₁, R'₂, R₄, R₅, X, Rx and Hal have the meanings indicated above,
which products of formulae (IV), (V), (IX) and (XI), if necessary or if desired, in order to obtain the products of formula (I) as defined in claim 1, can be subjected to any one or more of the following reactions, in any order:
a) if appropriate conversion of the >C=S group which can be represented by >C=X to a >C=O group,
b) release of the OH radical which can be carried by R'₃,
c) esterification or etherification reaction of the OH radical which can be carried by R₃,
d) elimination reaction of the optional protective groups,
e) if appropriate the action of an esterification, amidification or salification agent,
f) resolution reaction of the racemic forms,
said products of formula (I) thus obtained being in all possible racemic, enantiomeric and diastereoisomeric isomer forms.

4. As medicaments, the products of formula (I) as defined in claims 1 and 2, said products of formula (I) being in all possible racemic, enantiomeric and diastereoisomeric isomer forms, as well as the addition salts with pharmaceutically acceptable mineral and organic acids or with mineral and organic bases, of said products of formula (I).

5. As medicaments, the following products as defined in claims 1 and 2:
- carbonate of 4-(3-(4-cyano-3-(trifluoromethyl)phenyl)-5,5-dimethyl-2,4-dioxo-1-imidazolidinyl)butyl and of 1-methylethyl,
- 4-(4,4-dimethyl-2,5-dioxo-3-(4-nitrooxybutyl)-1 imidazolidinyl)-2-(trifluoromethyl)benzonitrile, said products of formula (I) being in all possible racemic, enantiomeric and diastereoisomeric isomer forms, as well as the addition salts with pharmaceutically acceptable mineral and organic acids or with mineral and organic bases, of said products of formula (I).

6. The pharmaceutical compositions containing, as active ingredient, at least one of the medicaments as defined in any one of claims 4 and 5.

7. As industrial products, the products of formulae (IV) and (V) 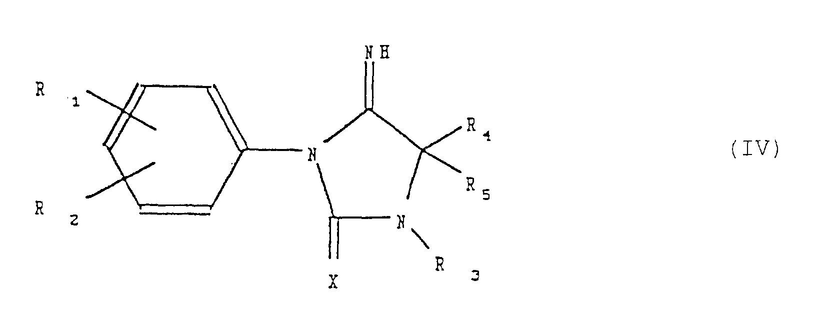 in which R₁, R₂, R₃, R₄ and R₅ have the meanings indicated in claim 1 and X represents an oxygen or sulphur atom 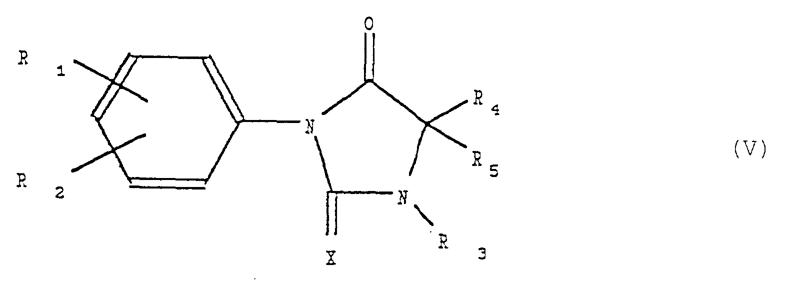 in which R₁, R₂, R₃, R₄ and R₅ have the meanings indicated in claim 1 and X represents a sulphur atom

8. Use of the products of formula (I) as defined in claims 1 and 2, for the preparation of pharmaceutical compositions intended for the treatment of adenomas and neoplasias of the prostate as well as of benign hypertrophia of the prostate, alone or in combination with analogues of LHRH, for the treatment of cutaneous affections such as acne, hyperseborrhea, alopecia or hirsutism or in diagnostics in medical imaging.

9. Use of the products of formula (I) as defined in claims 1 and 2, in which R₃ represents a radical substituted by the nitrooxo radical, for the preparation of pharmaceutical compositions intended for the treatment of cutaneous affections such as acne, alopecia or hirsutism.
